# EUROPEAN PATENT APPLICATION

(11) **EP 2 605 030 A1**
(43) Date of publication of application: **19.06.2013**
(21) Application number: 12195876.3
(22) Date of filing: 06.12.2012
(51) Int. Cl.: G01R 33/54

(54) **Method of capturing magnetic resonance image and magnetic resonance imaging apparatus using the same**

(30) Priority: 16.12.2011 KR 20110136563
(71) Applicant: Samsung Electronics Co., Ltd, Gyeonggi-do 443-742 (KR)
(72) Inventor: Oh, Keum-yong, Gyeonggi-do (KR); Lee, Jun-ki, Gyeonggi-do (KR); Lee, In-won, Incheon (KR)
(74) Representative: D'Halleweyn, Nele Veerle Trees Gertrudis

(57) **Abstract**

A method captures a magnetic resonance image for increasing convenience of a user, and a magnetic resonance imaging (MRI) apparatus uses the method, which includes: obtaining shape information of a subject; adjusting a field of view (FoV) according to the shape information of the subject; setting a K-space corresponding to the adjusted FoV; and capturing the magnetic resonance image by using the set K-space.

## Description

### CROSS-REFERENCE TO RELATED PATENT APPLICATION

### AND CLAIM OF PRIORITY

This application claims, pursuant to 35 U.S.C. 119(a), priority to and the benefit of the earlier filing date of a Korean Patent Application No. 10-2011-0136563, filed on December 16, 2011, in the Korean Intellectual Property Office, the disclosure of which is incorporated herein in its entirety by reference.

### BACKGROUND OF THE INVENTION

### 1. Field of the Invention

The present invention relates to a method of capturing a magnetic resonance image and a magnetic resonance imaging apparatus using the method.

### 2. Description of the Related Art

Magnetic resonance imaging (MRI) apparatuses, which are used to obtain an image of a subject by using a magnetic field, are widely used to accurately diagnose diseases since MRI apparatuses show highly detailed and/or stereoscopic images of bones, disks, joints, nerves, ligaments, etc. at a desired angle.

A photographing time of an MRI apparatus is changed according to a resolution setting and a photographing area. In particular, the number of pixels of an image increases according to the size of an area to be photographed, and the photographing time increases in proportion to the number of pixels of the image.

In the prior art, the typical photographing time of an MRI apparatus is about 1 hour. In general, an MRI apparatus is constructed as a long and narrow tube (hereinafter, referred to as an "MRI photographing tube"). Thus, a patient, for whom a magnetic resonance image is to be captured, should not move during the photographing time in an MRI photographing tube. Thus, a seriously-ill patient or a claustrophobic patient has difficulty in using an MRI apparatus, and increasing the photographing time generally increases a patient's feeling of boredom and inconvenience.

Thus, an MRI apparatus and a method that can reduce the photographing time are required.

After capturing a magnetic resonance image, image processing is performed to eliminate noise existing in the magnetic resonance image or to make a medical doctor's diagnosis easier.

A time that is required for the image processing increases in proportion to the size of a photographing area and the number of pixels of an image to be captured. Thus, it is necessary to enable a user, i.e., a patient, to more conveniently use an MRI apparatus by minimizing the time required for the image processing.

### SUMMARY OF THE INVENTION

The present invention provides a method of capturing a magnetic resonance image by using a magnetic resonance imaging (MRI) apparatus, which increases the convenience of a user.

The present invention also provides an MRI apparatus for increasing the convenience of a user.

The present invention also provides a method of capturing a magnetic resonance image by using an MRI apparatus, which decreases feelings of confinement and boredom of a user during magnetic resonance image capturing.

The present invention also provides an MRI apparatus for decreasing feelings of confinement and boredom of a user during magnetic resonance image capturing.

The present invention also provides a method of capturing a magnetic resonance image by using an MRI apparatus, which decreases a time that is required during magnetic resonance image capturing and a processing time of a magnetic resonance image.

The present invention also provides an MRI apparatus for decreasing a time that is required during magnetic resonance image capturing and a processing time of a magnetic resonance image.

According to an aspect of the present invention, there is provided a method of capturing a magnetic resonance image by using a magnetic resonance imaging (MRI) apparatus, the method including: obtaining shape information of a subject; adjusting a field of view (FoV) according to the shape information of the subject; setting a K-space corresponding to the adjusted FoV; and capturing the magnetic resonance image by using the set K-space.

The method may further include storing location information that is used to restore the captured magnetic resonance image to a magnetic resonance image corresponding to the size of an original FoV.

The storing of the location information may include storing coordinate information of the captured magnetic resonance image which is based on the magnetic resonance image corresponding to the size of the original FoV.

The coordinate information may include at least one coordinate value of an outer boundary of the captured magnetic resonance image.

The location information may include information about a center point and a radius of the captured magnetic resonance image.

The location information may include information about a size ratio of the captured magnetic resonance image to the original magnetic resonance image corresponding to the size of the original FoV.

The method may further include restoring the captured magnetic resonance image to have the same size as the original magnetic resonance image corresponding to the size of the original FoV by using the location information.

The adjusting of the FoV may include setting a region having a predetermined margin to a boundary of the subject as the FoV.

The adjusting of the FoV may include obtaining the boundary of the subject for each of a plurality of slices based on the shape information and setting the FoV by adding the predetermined margin to the boundary of the subject for each slice.

The adjusting of the FoV may include estimating a boundary of the subject in a current slice based on the boundary of the subject in a previous slice and setting the FoV of the current slice by using the estimated boundary of the subject.

The obtaining of the shape information of the subject may include obtaining a scout image of the subject.

According to an aspect of the present invention, there is provided a magnetic resonance imaging (MRI) apparatus including: a control unit for obtaining shape information of a subject, adjusting a field of view (FoV) according to the obtained shape information of the subject, and setting a K-space corresponding to the adjusted FoV; and an image capturing unit for capturing a magnetic resonance image of the subject according to the set K-space.

The MRI apparatus may further include a storage unit for storing location information that is used to restore the captured magnetic resonance image to an original magnetic resonance image corresponding to the size of an original FoV.

The storage unit may store coordinate information of the captured magnetic resonance image which is based on the magnetic resonance image corresponding to the size of the original FoV.

The coordinate information may include at least one coordinate value of an outer boundary of the captured magnetic resonance image.

The storage unit may store the location information including information about center coordinates and a radius of the captured magnetic resonance image.

The storage unit may store the location information including information about a size ratio of the captured magnetic resonance image to the original magnetic resonance image corresponding to the size of the original FoV.

The control unit may restore the captured magnetic resonance image to have the same size as the original magnetic resonance image corresponding to the size of the original FoV by using the location information.

The control unit may set a region having a predetermined margin around a boundary of the subject as the FoV.

The control unit may obtain the boundary of the subject for each of a plurality of slices based on the shape information and may set the FoV by adding the predetermined margin to the boundary of the subject for each slice.

The control unit may estimate a boundary of the subject in a current slice based on the boundary of the subject in a previous slice and may set the FoV of the current slice by using the estimated boundary of the subject.

The control unit may obtain a scout image of the subject as the shape information of the subject.

The control unit may receive an image of the subject and may obtain the shape information of the subject from the received image.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram illustrating a magnetic resonance imaging (MRI) apparatus according to an exemplary embodiment of the present invention;
FIG. 2 is a diagram illustrating an MRI apparatus according to another exemplary embodiment of the present invention;
FIG. 3 is a diagram illustrating a magnetic resonance image;
FIG. 4 is a diagram for explaining a field of view (FoV) adjustment operation;
FIGS. 5A-5C are diagrams for explaining a magnetic resonance image captured according to an adjusted FoV;
FIG. 6 is a diagram for explaining location information that is stored in a storage unit illustrated in FIG. 2;
FIGS. 7A-7C are diagrams for explaining a process of restoring a magnetic resonance image captured by using an adjusted FoV to a magnetic resonance image corresponding to the size of an original FoV; and
FIG. 8 is a flowchart illustrating a method of capturing a magnetic resonance image, according to the exemplary embodiment of the present invention.

### DETAILED DESCRIPTION OF THE INVENTION

Hereinafter, preferred embodiments of the present invention will be described with reference to the accompanying drawings. The same reference numbers are used throughout the drawings to refer to the same or like parts. In the following description, a detailed explanation of known related functions and constructions may be omitted to avoid unnecessarily obscuring the subject matter of the present invention. This invention may, however, be embodied in many different forms and should not be construed as limited to the exemplary embodiments set forth herein. Also, terms described herein, which are defined considering the functions of the present invention, may be implemented differently depending on user and operator's intention and practice. Therefore, the terms should be understood on the basis of the disclosure throughout the specification. The principles and features of this invention may be employed in varied and numerous embodiments without departing from the scope of the invention.

Furthermore, although the drawings represent exemplary embodiments of the invention, the drawings are not necessarily to scale and certain features may be exaggerated or omitted in order to more clearly illustrate and explain the present invention.

A magnetic resonance imaging (MRI) apparatus forms a main magnetic field by using a permanent magnet such as an annular-shaped magnet having a central bore in which a subject, such as a patient, is positioned, and forms a gradient magnetic field in an X, Y, or Z direction by using a gradient coil. A magnetic resonance image is formed by using a radio frequency (RF) signal that is received from the generated magnetic fields. In this case, the permanent magnet may be referred to as a main magnet, and the gradient coil may be referred to as a secondary magnet.

The MRI apparatus sets a field of view (FoV) for obtaining an MR image of the subject, or at least a portion of the subject, in the central bore, and forms the gradient magnetic field by turning on or off a switch for the gradient magnetic field in the X, Y, or Z direction with respect to the set FoV. The number of drivings of the gradient coil and a driving time of the gradient coil are proportional to the size of an area of the FoV.

In a method of capturing a magnetic resonance image and an MRI apparatus according to exemplary embodiments of the present invention described herein, by setting an optimized FoV according to a shape of the subject, that is, an object of magnetic resonance image capturing, the number of drivings of a gradient coil and a driving time of the gradient coil may be reduced, and thus a magnetic resonance image may be quickly captured. Thus, feelings of confinement and boredom of a user, i.e., a patient, during magnetic resonance image capturing may be reduced. Hereinafter, exemplary embodiments of the present invention will be described in detail with reference to the accompanying drawings.

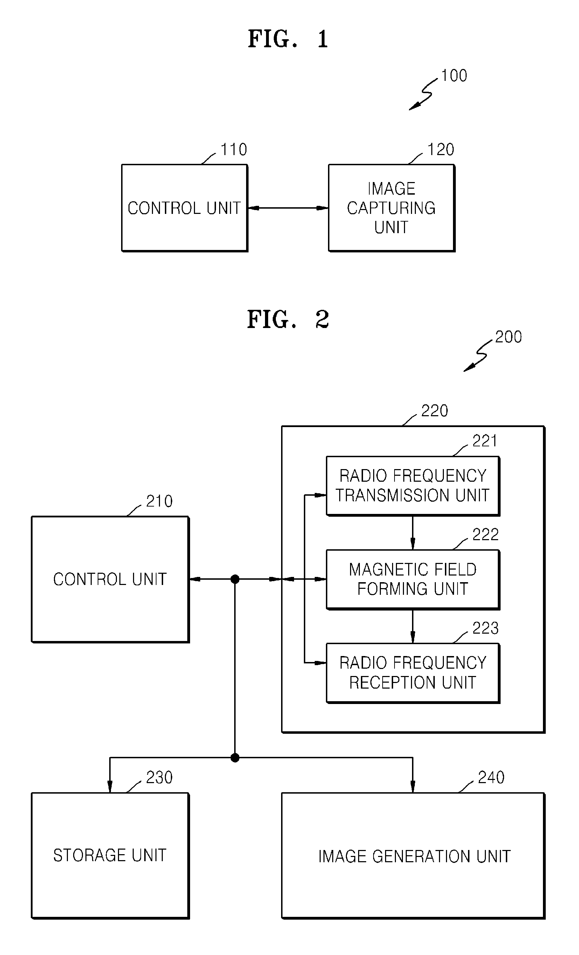

FIG. 1 is a block diagram illustrating an MRI apparatus 100 according to an exemplary embodiment of the present invention.

Referring to FIG. 1, the MRI apparatus 100 includes a control unit 110 and an image photographing unit 120.

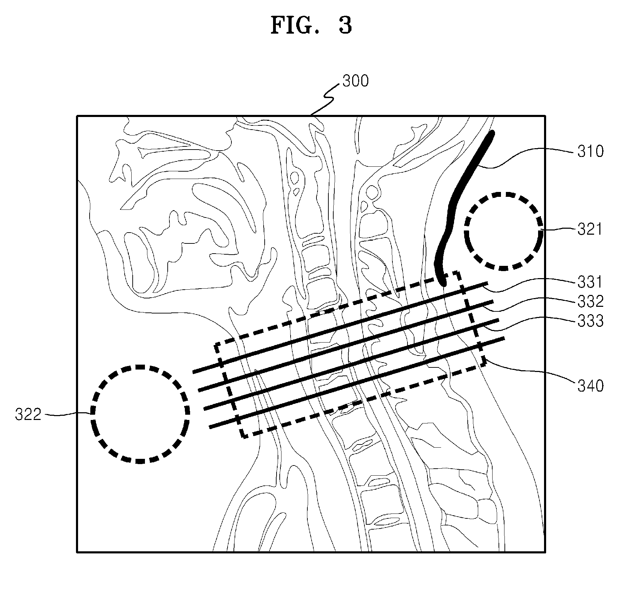

The control unit 110 obtains shape information of a subject and adjusts an FoV according to the obtained shape information of the subject using known automated image identification, classification, and extraction methods using a computer and image processing for obtaining the shape information including the black-gray-and-white images from the black background of an overall image, as shown in FIG. 3, as well as for adjusting the FoV. Then, the control unit 110 sets a K-space corresponding to the adjusted FoV using known automated image identification and classification, and extraction methods using a computer and image and data processing for setting the K-space. Hereinafter, an FoV before adjusting is referred to an original FoV.

The subject is a body portion of a user for which a magnetic resonance image is to be captured. The term "FoV" is defined herein to be the size of an area of interest in a photographing and imaging process. The K-space is a set of raw data by which an image may be formed. The number of drivings of a gradient coil and the number of phase encodings are changed according to the size of the K-space.

The image photographing unit 120 includes a gradient coil (not shown), and photographs the subject to capture a magnetic resonance image thereof according to the K-space adjusted by the control unit 110 by driving the gradient coil.

FIG. 2 is a diagram illustrating an MRI apparatus 200 according to another exemplary embodiment of the present invention.

Referring to FIG. 2, the MRI apparatus 200 includes a control unit 210 and an image capturing unit 220. The control unit 210 and the image capturing unit 220 correspond to the control unit 110 and the image photographing unit 120 of FIG. 1, respectively. Thus, a repeated description will be omitted below. In addition, the MRI apparatus 200 may further include at least one of a storage unit 230 and an image generation unit 240 for outputting an MR image, for example, on a display. The image capturing unit 220 includes a radio frequency transmission unit 221, a magnetic field forming unit 222, and a radio frequency reception unit 223.

The control unit 210 obtains shape information of a subject and adjusts an FoV according to the obtained shape information of the subject.

Generally, in magnetic resonance image capturing, a primary image of the subject is generated by previously photographing the subject overall and roughly, and then precise magnetic resonance image capturing is performed by defining a predetermined portion in the generated primary image. For example, a medical doctor photographs the head of a patient to capture a primary image, and may perform precise photographing again with respect to a portion of the head in which it is determined that there is an abnormality in the primary image, for example, the occipital lobe. The primary image may be a scout image that is a location confirmation image that is used for setting a location of a diagnosis portion in detail.

The control unit 210 may obtain a scout image as the shape information of the subject, and may adjust the FoV according to the shape information of the subject appearing in the obtained scout image. In addition, the control unit 210 may receive the shape information from another source, such as an outside or external source. In addition, the control unit 210 may obtain all images captured by photographing the same subject using MRI or other imaging methods, for example, X-ray images from X-ray photography of the same subject, and then may obtain the shape information of the subject from the obtained images. Alternatively, when photographing the subject by using a 3-dimensional (3-D) camera, the control unit 210 may receive profile data obtained by the 3-D camera as the shape information of the subject. In this case, the 3-D camera may be disposed inside or outside of the MRI apparatus 200.

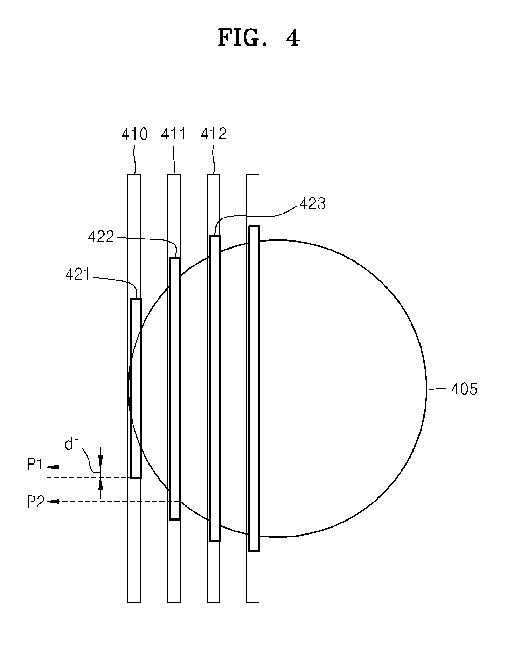

An FoV adjustment operation of the control unit 210 is described in detail with reference to FIGS. 3 and 4 below.

FIG. 3 is a diagram illustrating a magnetic resonance image. In particular, FIG. 3 illustrates a scout image 300.

The scout image 300 is an image. such as a preliminary image or a test image, obtained by photographing a subject as a whole, and the shape of the subject may obtained by using the scout image 300. In particular, the scout image 300 is divided into an image of a human body, i.e., the subject, and black colored portions 321 and 322 of the scout image 300 independent of the subject, based on an outermost boundary 310 of the subject. Hereinafter, a case in which the scout image 300 is shape information of the subject obtained by the control unit 210 of FIG. 2 is described as an example.

The MRI apparatus 200 may set a specific diagnosis portion 340 in the scout image 300. In particular, the control unit 210 sets the specific diagnosis portion 340 according to a selection of an operator of the MRI apparatus 200, for example, a medical doctor, and performs precise magnetic resonance image capturing with respect to the specific diagnosis portion 340. The control unit 210 slices the specific diagnosis portion 340 at a predetermined interval or spacing in a predetermined direction and then MR photographs the sliced area. In particular, the control unit 210 may control the image capturing unit 220 to capture magnetic resonance images of example sliced areas or slices 331, 332, and 333 within the specific diagnosis portion 340 illustrated in FIG. 3.

Referring to FIG. 3, the control unit 210 may extract the boundary 310 of the subject from the scout image 300, and may adjust the FoV by using the extracted boundary 310 using known automated image identification, classification, and extraction methods using a computer and image processing for obtaining the shape information including the black-gray-and-white images from the black background of an overall image, as shown in FIG. 3.

In particular, the control unit 210 may obtain the boundary 310 of the subject for each of the slices 331, 332, and 333 to be photographed, based on the shape information of the subject, for example, the scout image 300, and may set the FoV by adding a predetermined margin to the boundary 310 of the subject for each of the slices 331, 332, and 333. For example, the predetermined margin may include a section of the black colored image portions 321, 322 having a width of at most about 5 millimeters perpendicular to and extending from any portion of the outer areas of the slices 331, 332, 333, to provide contrast between the inside and outside portions of the subject for improved image processing.

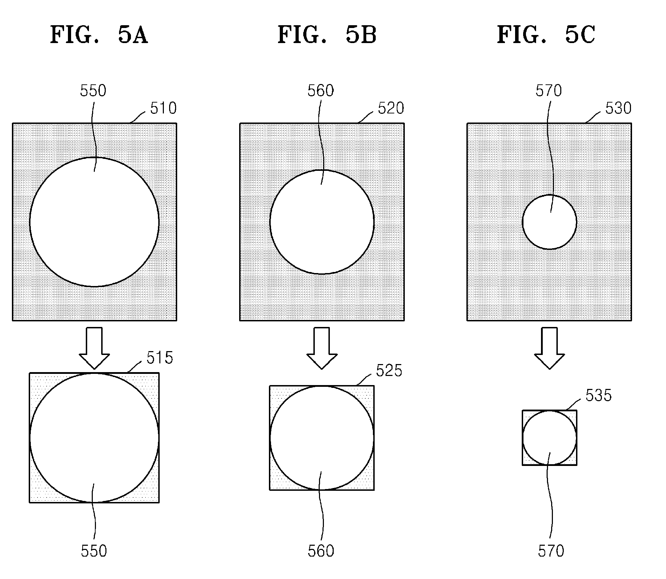

The FoV may then be set as a minimal circle having a radius or a minimal square with an inscribed circle having a radius, with the minimal circle or inscribed circle encompassing or bounding the entire shape information of the subject including the boundary and any predetermined margin, as described in greater detail in connection with FIGS. 5A-7C.

FIG. 4 is a diagram for explaining an FoV adjustment operation.

In FIG. 4, FoVs 410, 411, and 412 are shown which indicate FoVs that are used in a general MRI apparatus to photograph slice cross sections. In addition, FoVs 421, 422, and 423 indicate FoVs that are used in the MRI apparatuses according to the exemplary embodiments of the present invention to photograph slice cross sections. For example, the FoV 410 and the FoV 421 are FoVs that are applied to photograph the same slice cross section. In addition, the FoV 411 and the FoV 422 are FoVs that are applied to photograph the same slice cross section.

Referring to FIG. 4, the control unit 210 may set an FoV optimized to a subject 405, based on shape information of the subject 405. For illustrative purposes only to describe the present invention herein, the subject 405 is generally represented in cross-section in the drawings as a circle, but it is understood that the cross-section of any portion of a subject 405 may be irregularly shaped. For example, the control unit 210 may set a region having a predetermined margin d1 from a boundary of the subject 405, for example, the boundary 310 illustrated in FIG. 3, as the FoV. The predetermined margin d1 may be set in consideration of an error range of the boundary 310. Otherwise, the predetermined margin d1 may be set in consideration of an area that is sliced, that is, a photographing cross section area of the subject 405. For example, the predetermined margin d1 may be a predetermined fraction of the error range and/or the size of the photographing cross section area, or may be a predetermined function of the error range and/or the area. Alternatively, the predetermined margin d1 may be set by a user, and may be set by the control unit 210.

Referring to FIG. 4, the control unit 210 may set the FoVs 421, 422, and 433 based on the boundary 310 of the subject 405, as illustrated in FIGS. 3-4. Since an MRI apparatus precisely photographs a human body portion in which it is supposed that there is a disease therein, generally, an interval for slicing the magnetic resonance image is set to a relatively small value. In addition, the boundary 310 of the human body that is a photographing object of an MRI apparatus is not suddenly changed. Thus, a boundary P2 in a next slice (for example, the FoV 422) may be estimated by using a gradient value of a boundary P1 in a previous slice (for example, the FoV 421) using a known estimation method.

Thus, the control unit 210 may estimate the boundary P2 of the subject 405 in a current slice (for example, the FoV 422) based on the boundary P1 of the subject 405 in a previous slice (for example, the FoV 421), and then may set an FoV of the current slice by using the estimated boundary of the subject 405.

A conventional MRI apparatus in the prior art may set or use uniform FoVs. For example, a conventional MRI apparatus photographs a subject to capture a magnetic resonance image thereof by setting the FoVs 410, 411, and 412 for each slice. As shown in FIG. 4, each of the FoVs 410, 411, 412 have equal and fixed sizes. A time that is required for magnetic resonance image capturing increases in proportion to the size of an FoV. In addition, a time that is required for signal processing such as eliminating noise of a captured magnetic resonance image and adjusting brightness thereof increases in proportion to the size of an FoV.

As stated above, the MRI apparatuses 100 and 200 according to the above exemplary embodiments of the present invention optimize an FoV according to a shape of a subject to set the FoV and thus may reduce a time that is required for magnetic resonance image capturing. Thus, the use of the MRI apparatuses 100 and 200 may decrease feelings of confinement and boredom of a patient during magnetic resonance image capturing.

In addition, the MRI apparatuses 100 and 200 of the present invention may eliminate a time that is required for photographing a region other than the subject 405, compared to when capturing a magnetic resonance image by using the conventional uniform FoVs 410, 411, and 412 used in the prior art. For example, referring to FIG. 3, the black colored regions 321, 322 are not part of the FoVs 421, 422, 423 in FIG. 4, and so are not processed in the MRI photographing and image processing of the present invention. In addition, the MRI apparatuses 100 and 200 of the present invention may reduce a time that is required for signal processing of a captured magnetic resonance image and thus may increase convenience of a user, for example, a patient.

In each exemplary embodiment of the present invention, each control unit 110, 210 sets a K-space corresponding to an adjusted FoV, such as the FoVs 421, 422, 423, in a manner known in the art. An FoV before being adjusted may be referred to as an original FoV. For example, the FoVs 410, 411, and 412 in the prior art of which sizes have been set to a fixed or maximum size may be original FoVs.

When capturing a magnetic resonance image by applying an original FoV (for example, the original FoV 410) the K-space that is applied to capture the magnetic resonance image may have a matrix size of 128*128.

For example, when an FoV adjusted according to the boundary 310 of the subject 405, for example, the FoV 422, has a size of a quarter of the FoV 411 set to the maximum size, the K-space may have a size of 64*64, which is one-quarter of the size of the original 128*128 matrix. Thus, a time that is required for the image capturing unit 220 to capture a magnetic resonance image in a predetermined slice according to a control of the control unit 210 may be reduced to a quarter of a time that is required in the prior art when capturing a magnetic resonance image having a maximum resolution.

The image capturing unit 220 captures a magnetic resonance image of a subject according to a set K-space, according to a control of the control unit 210. A process of capturing a magnetic resonance image by using the set K-space is described with reference to FIGS. 5A-5C below.

As stated above, the image capturing unit 220 may include the radio frequency transmission unit 221, the magnetic field forming unit 222, and the radio frequency reception unit 223.

The radio frequency transmission unit 221 generates a predetermined frequency signal, i.e., a radio frequency (RF) signal, used for instructing the annular magnet of the MRI apparatus to form a magnetic field, and then outputs the generated RF signal to the magnetic field forming unit 222. In particular, the radio frequency transmission unit 221 may transmit a modulated and amplified RF signal to the magnetic field forming unit 222, which includes a photographing space for a subject, that is, the MRI bore of the annular magnet in which the subject is positioned. The radio frequency transmission unit 221 generates the RF signal according to the K-space set by the control unit 210 and then outputs the generated RF signal.

The magnetic field forming unit 222 generates a magnetic resonance (MR) signal according to the RF signal generated in the radio frequency transmission unit 221. The magnetic field forming unit 222 may include the MRI bore (not shown) including a main magnet and a secondary magnet. The secondary magnet may include a gradient coil (not shown). The magnet field forming unit 222 drives the gradient coil depending on the K-space set by the control unit 210.

The radio frequency reception unit 223 receives a radio frequency signal output from the magnetic field forming unit 222 and then transmits the received radio frequency signal to the image generation unit 240.

The image generation unit 240 generates a magnetic resonance image by using the radio frequency signal received from the radio frequency reception unit 223.

FIGS. 5A-5C are diagrams for explaining a magnetic resonance image captured according to an adjusted FoV used in the present invention.

Referring to FIGS. 5A-5C, images 510, 520, and 530 indicate magnetic resonance images captured in the image capturing unit 220 in a case in which an FoV has not been adjusted. That is, the images 510, 520, and 530 indicate magnetic resonance images captured by applying an original FoV. Images 515, 525, and 535 indicate magnetic resonance images captured in the image capturing unit 220 in a case in which an FoV has been adjusted by the control unit 210. In this case, each of the images 515, 525, and 535 determines a range of MRI photographing determined by the K-space stated above.

Using the FoVs 410, 411, 412 of FIG. 4, the image 510 indicates a magnetic resonance image of a subject, represented in FIGS. 5A-5C by the illustrative cross sections 550, 560, 570, captured by applying the original FoV 412, and the image 520 indicates a magnetic resonance image of the subject 560 captured by applying the original FoV 411. The image 530 indicates a magnetic resonance image of the subject 570 captured by applying the original FoV 410.

Using the FoVs 421, 422, 423 of FIG. 4 according to the present invention, the image 515 indicates a magnetic resonance image of the subject 550 captured by applying the adjusted FoV 423, and the image 525 indicates a magnetic resonance image of the subject 560 captured by applying the adjusted FoV 422. The image 535 indicates a magnetic resonance image of the subject 570 captured by applying the adjusted FoV 421. In FIGS. 5A-5C, a case where the predetermined margin d1 stated above is set to or equal to zero is illustrated as an example.

The storage unit 230 may store location information that is used to restore a magnetic resonance image (for example, the image 515) captured by applying an adjusted FoV to a magnetic resonance image (for example, the image 510) corresponding to the size of an original FoV.

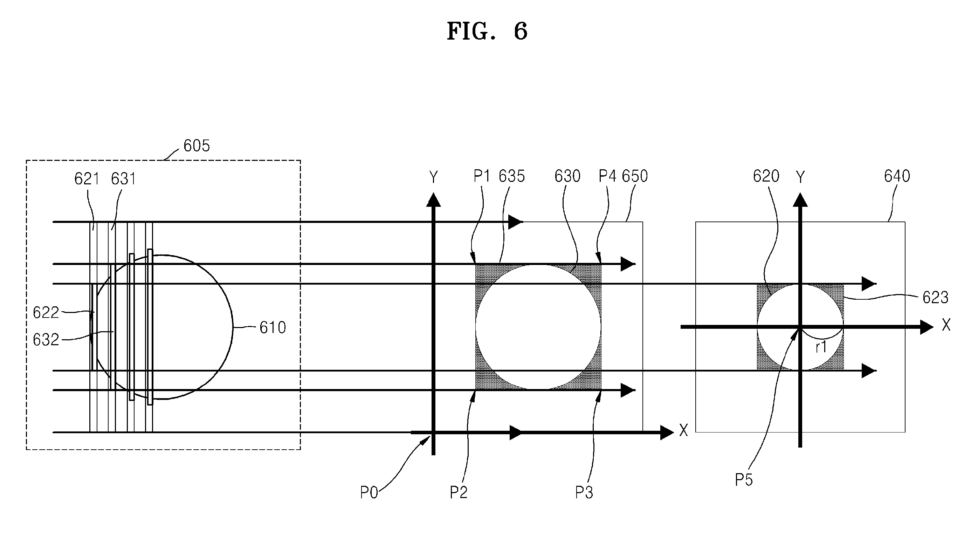

In particular, the control unit 210 may generate the location information that is used to restore the size of a magnetic resonance image (for example, the image 515) captured by applying an adjusted FoV so that the magnetic resonance image (for example, the image 515) captured by applying the adjusted FoV has the same size as a magnetic resonance image (for example, the image 510) captured by applying an original FoV. The control unit 210 controls the storage unit 230 to store the generated location information in the storage unit 230. That is, the control unit 210 may restore the size of a magnetic resonance image (for example, the image 515) captured by applying the adjusted FoV 423 to the size of a magnetic resonance image (for example, the image 510) corresponding to an original FoV (for example, the original FoV 412). The location information is described in detail with reference to FIG. 6 below.

FIG. 6 is a diagram for explaining the location information that is stored in the storage unit 230 illustrated in FIG. 2.

Referring to FIG. 6, a block 605 corresponds to the slicing of a subject 405 as shown in FIG. 4, oriented in the y-z plane. In particular, FoVs 621, 622, 631, and 632 of the block 605 correspond to the FoVs 410, 421, 411, and 422 of FIG. 4, respectively, and a subject 610 of the block 605 corresponds to the subject 405 of FIG. 4. Images 635 and 623 correspond to the images 525 and 535 of FIGS. 5B-5C, respectively, with the images 623, 635 of FIG. 6 oriented in the x-y plane, corresponding to the block 605 in the y-z plane of FIG. 6. Cross sections of subjects 630 and 620 correspond to cross sections of subjects 560 and 570 of FIG. 5B-5C, respectively. Thus, a repeated description will be omitted.

The control unit 210 may generate coordinate information of a magnetic resonance image (for example, the image 635) based on a magnetic resonance image (for example, an original image 650) corresponding to the size of an original FoV. Otherwise, the control unit 210 may generate coordinate information corresponding to the magnetic resonance image 635 captured by adjusting an FoV for the magnetic resonance image 635 based on an original FoV for the original image 650. That is, the control unit 210 may generate the location information including the coordinate information.

In particular, as shown in FIG. 6, the control unit 210 sets coordinates (for example, X and Y coordinates) based on a predetermined point P0 which is reference or origin point of the magnetic resonance image 650 corresponding to the size of the original FoV, and generates information that may indicate a location of the subject 630 in the set coordinates.

The coordinate information may include at least one of coordinate values P1, P2, P3, and P4 of an outer boundary of the magnetic resonance image 635. That is, the at least one of the coordinate values P1, P2, P3, and P4 may be generated as the coordinate information of the magnetic resonance image 635 captured by applying the adjusted FoV.

The storage unit 230 may store coordinate information of a magnetic resonance image (for example, the image 635) captured in a magnetic resonance image (for example, the image 650) corresponding to the size of an original FoV. As stated above, the coordinate information is generated by the control unit 210.

The control unit 210 may generate location information that includes information about the center coordinates P5 and a radius r1 of the magnetic resonance image 623 captured applying an adjusted FoV. In this case, the storage unit 230 may store the location information that includes the information about the center point P5 and the radius r1. It is understood that the radius r1 may refer to the radius of an inscribed circle associated with the square-shaped image 623 in which the image of the subject 620 and/or its associated FoV 622 is positioned. Since the subjects 610, 620, 630 are represented in cross-section by circles, for illustrative purposes only, and so such cross-sections may be irregularly shaped, the radius r1 may not correspond to a radius of the subjects 610, 620, 630.

Alternatively, the control unit 210 may generate location information that includes information about a size ratio of a magnetic resonance image (for example, the image 635) captured by applying an adjusted FoV to a magnetic resonance image (for example, the image 650) corresponding to the size of an original FoV. In this case, the storage unit 230 may store the location information that includes the information about the ratio. In particular, if the size ratio of the magnetic resonance image 635 captured by applying the adjusted FoV to the magnetic resonance image 650 corresponding to the size of the original FoV is 1:2, the control unit 210 may generate information about the size ratio 1:2 as the location information.

The control unit 210 may restore a captured magnetic resonance image (for example, the image 635) such that the captured magnetic resonance image (for example, the image 635) has the same size as a magnetic resonance image (for example, the image 650) corresponding to the size of an original FoV. When a medical doctor diagnoses a plurality of magnetic resonance images corresponding to a plurality of slices, it is convenient for the medical doctor to use magnetic resonance images having the same size. Thus, the control unit 210 may perform a size restoration operation.

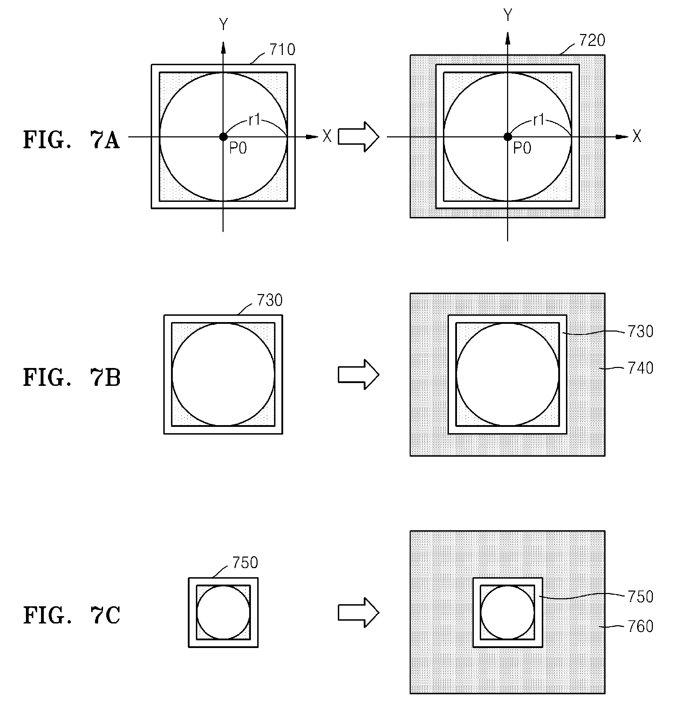

The size restoration operation of the control unit 210 is described in detail with reference to FIGS. 7A-7C below.

FIGS. 7A-7C are diagrams for explaining a process of restoring a magnetic resonance image captured by using an adjusted FoV to a magnetic resonance image corresponding to the size of an original FoV.

Referring to FIGS. 7A-7C, images 710, 730, and 750 indicate magnetic resonance images captured by applying an adjusted FoV. Images 710, 730, and 750 correspond to the images 515, 525, and 535 illustrated in FIGS. 5A-5C, respectively.

In FIGS. 7A-7C, a case where location information that is stored in the storage unit 230 includes information about a center point P0 and a radius r1 of a captured magnetic resonance image (for example, the image 710) is illustrated as an example. Referring to FIG. 7A, control unit 210 may increase the size of the magnetic resonance image 710 captured by applying the adjusted FoV, according to a magnetic resonance image 720, by matching the magnetic resonance image 710 to the center coordinates P0 of the magnetic resonance image 720 corresponding to the size of the original FoV. In particular, as illustrated in FIGS. 7A-7C, the size of the magnetic resonance image 710 may be adjusted to that of the magnetic resonance image 720 while equally maintaining the image resolutions of subjects included in the magnetic resonance images 710 and 720.

Referring to FIGS. 7B-7C, respectively, images 730 and 750 may be restored so that the sizes of the images 730 and 750 become equal to the sizes of the images 740 and 760, respectively, by using the location information stored in the storage unit 230.

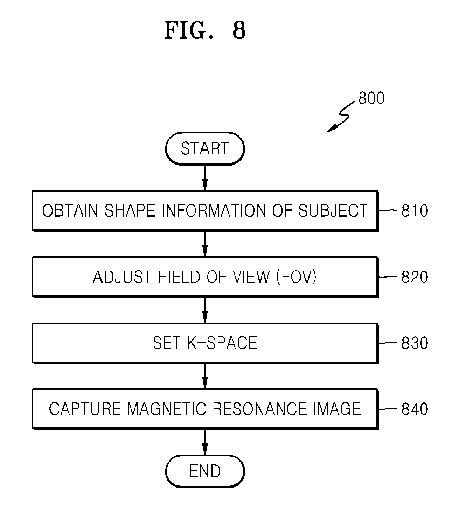

FIG. 8 is a flowchart illustrating a method 800 of capturing a magnetic resonance image, according to the exemplary embodiment of the present invention.

Referring to FIG. 8, the method 800 is a method of capturing a magnetic resonance image by using the magnetic resonance imaging apparatuses according to the above exemplary embodiments of the present invention. The method 800 has the same features as the operations of the magnetic resonance imaging apparatuses according to the above exemplary embodiments of the present invention, which have been described with reference to FIGS. 1 through 7C. Thus, a repeated description will be omitted. Hereinafter, the method 800 is described with respect to the magnetic resonance imaging apparatus 200 illustrated in FIG. 2.

In step 810, shape information of a subject is obtained. Step 810 may be performed in the control unit 210. In particular, in step 810, a scout image of the subject may be obtained as the shape information of the subject. Alternatively, the shape information may be obtained from all images captured from the subject.

In step 820, a FoV is adjusted according to the shape information obtained in step 810. Step 820 may be performed in the control unit 210.

In step 820, with respect to a slice, a region having a predetermined margin from a boundary of the subject may be set as the FoV. That is, based on the shape information obtained in step 810, the boundary of the subject for each slice may be obtained, and the FoV may be set by adding the predetermined margin to the boundary of the subject for each slice.

In addition, in step 820, the boundary of the subject in a current slice may be estimated based on the boundary of the subject in a previous slice, and the FoV of the current slice may be set using the estimated boundary of the subject.

A K-space is set in step 830 corresponding to the FoV set in step 820, which may be performed in the control unit 210.

A magnetic resonance image is captured in step 840 by using the K-space set in step 830. Step 840 may be performed in the image capturing unit 220 according to a control of the control unit 210.

In addition, the method 800 may further include storing location information that is used to restore the magnetic resonance image captured in step 840 to a magnetic resonance image corresponding to the size of an original FoV. The storing of the location information may be performed after step 840.

The storing of the location information may include storing coordinate information of the magnetic resonance image captured in step 840 which is based on the magnetic resonance image corresponding to the size of the original FoV. The coordinate information may include at least one coordinate value of an outer boundary of the magnetic resonance image captured in step 840.

In addition, the storing of the location information may include storing location information that includes information about center coordinates and a radius of the magnetic resonance image captured in step 840.

Furthermore, the storing of the location information may include storing location information comprising information about a size ratio of the magnetic resonance image captured in step 840 to the magnetic resonance image corresponding to the size of the original FoV.

In addition, the method 800 may further include restoring the magnetic resonance image captured in step 840, by using the location information, so that the magnetic resonance image captured in step 840 has the same size as the magnetic resonance image corresponding to the size of the original FoV. In addition, feelings of confinement and boredom of a user during magnetic resonance image capturing may be minimized.

The above-described apparatus and methods according to the present invention can be implemented in hardware, firmware or as software or computer code that can be stored in a recording medium such as a CD ROM, a RAM, a ROM, a floppy disk, DVDs, a hard disk, a magnetic storage media, an optical recording media, or a magneto-optical disk or computer code downloaded over a network originally stored on a remote recording medium, a computer readable recording medium, or a non-transitory machine readable medium and to be stored on a local recording medium, so that the methods described herein can be rendered in such software that is stored on the recording medium using a general purpose computer, a digital computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein.

While the present invention has been particularly shown and described with reference to exemplary embodiments thereof, it will be understood by those of ordinary skill in the art that various changes in form and details may be made therein without departing from the spirit and scope of the present invention as defined by the following claims. Accordingly, the disclosed embodiments should be considered in an illustrative sense rather than a limiting sense. The scope of the present invention is defined not by the detailed description of the present invention but by the appended claims, and all differences within the scope will be construed as being included in the present invention.

## Claims

1. A method of capturing a magnetic resonance image by using a magnetic resonance imaging (MRI) apparatus, the method comprising:
obtaining shape information of a subject;
adjusting a field of view (FoV) according to the shape information of the subject;
setting a K-space corresponding to the adjusted FoV; and
capturing the magnetic resonance image by using the set K-space.

2. The method of claim 1, further comprising storing location information that is used to restore the captured magnetic resonance image to a magnetic resonance image corresponding to the size of an original FoV.

3. The method of claim 2, wherein the storing of the location information comprises storing coordinate information of the captured magnetic resonance image which is based on the magnetic resonance image corresponding to the size of the original FoV.

4. The method of claim 3, wherein the coordinate information comprises at least one coordinate value of an outer boundary of the captured magnetic resonance image.

5. The method of claim 2, wherein the location information comprises information about a center point and a radius of the captured magnetic resonance image.

6. The method of claim 2, wherein the location information comprises information about a size ratio of the captured magnetic resonance image to the magnetic resonance image corresponding to the size of the original FoV.

7. The method of claim 2, further comprising restoring the captured magnetic resonance image to have the same size as the magnetic resonance image corresponding to the size of the original FoV by using the location information.

8. The method of claim 1, wherein the adjusting of the FoV comprises setting a region having a predetermined margin to a boundary of the subject as the FoV.

9. The method of claim 8, wherein the adjusting of the FoV comprises obtaining the boundary of the subject for each of a plurality of slices based on the shape information and adjusting the FoV by adding the predetermined margin to the boundary of the subject for each slice.

10. The method of claim 1, wherein the adjusting of the FoV comprises estimating a boundary of the subject in a current slice based on the boundary of the subject in a previous slice and adjusting the FoV of the current slice by using the estimated boundary of the subject.

11. The method of claim 1, wherein the obtaining of the shape information of the subject comprises obtaining a scout image of the subject.

12. A magnetic resonance imaging (MRI) apparatus comprising:
a control unit for obtaining shape information of a subject, adjusting a field of view (FoV) according to the obtained shape information of the subject, and setting a K-space corresponding to the adjusted FoV; and
an image capturing unit for capturing a magnetic resonance image of the subject according to the set K-space.

13. The MRI apparatus of claim 12, further comprising a storage unit for storing location information that is used to restore the captured magnetic resonance image to a magnetic resonance image corresponding to the size of an original FoV.

14. The MRI apparatus of claim 13, wherein the storage unit stores coordinate information of the captured magnetic resonance image which is based on the magnetic resonance image corresponding to the size of the original FoV.

15. The MRI apparatus of claim 14, wherein the coordinate information comprises at least one coordinate value of an outer boundary of the captured magnetic resonance image.
